# EUROPÄISCHE PATENTSCHRIFT

(11) **EP 2 558 190 B1**
(45) Veröffentlichungstag und Bekanntmachung des Hinweises auf die Patenterteilung: **06.07.2016**
(21) Anmeldenummer: 10765635.7
(22) Anmeldetag: 15.10.2010
(51) Int. Cl.: B01F 3/08, B01F 5/06, B01F 15/02, A61K 8/22, A61Q 5/10, B01F 15/00

(54) **VERFAHREN ZUR HERSTELLUNG EINES FÄRBEMITTELS FÜR KERATINFASERN**
PROCESS FOR PREPARING A COLOURING COMPOSITION FOR KERATINOUS FIBRES
PROCÉDÉ DE FABRICATION D'UN COLORANT POUR FIBRES KERATINIQUES

(30) Priorität: 15.04.2010 DE 102010027824
(43) Veröffentlichungstag der Anmeldung: 20.02.2013
(73) Patentinhaber: Henkel AG & Co. KGaA, 40589 Düsseldorf (DE)
(72) Erfinder: JANßEN, Frank, 51103 Köln (DE); SCHÖPGENS, Jürgen, 41366 Schwalmtal (DE); WADLE, Armin, 40699 Erkrath (DE); FÖRSTER, Thomas, 40591 Düsseldorf (DE); CHEN, Lisa, 1060 Wien (AT)
(86) Internationale Anmeldenummer: PCT/EP2010/065504
(87) Internationale Veröffentlichungsnummer: WO 2011/141069

(56) Entgegenhaltungen:
- EP-A1- 2 062 616
- EP-A2- 0 132 511
- EP-A2- 0 152 761
- DE-A1- 1 617 825
- DE-A1- 1 801 518
- DE-A1- 2 359 399
- DE-A1- 3 843 892
- DE-A1- 4 133 957
- DE-A1- 4 234 887
- DE-A1- 4 440 957
- DE-A1- 19 543 988
- US-A- 3 548 562
- US-A1- 2006 002 965
- US-A1- 2006 164 913

## Beschreibung

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Die Herstellung dieser Färbemittel, insbesondere oxidativer Färbemittel oder Blondierpulver erfolgt in der Regel durch manuelle Vermischung vorgefertigter Wirkstoffzusammensetzungen, beispielsweise zweier oxidativer Färbemittel. Die manuelle Vermischung ist jedoch zeit- und arbeitsintensiv, da die Bestandteile vor der Vermischung beispielsweise eingewogen werden müssen. Bei der Verarbeitung fester Wirkstoffzusammensetzungen ist der Anwender zudem eventuell auftretenden Stäuben ausgesetzt.

Verfahren zur Herstellung von oxidativen Färbemitteln unter Einsatz von Vorrichtungen mit zwei voneinander getrennten Kammern werden in der europäischen Patentanmeldung EP 2 062 616 A1 sowie den US-amerikanischen Patentanmeldungen US 3 548 562 A und US 2006/002965 A1 beschrieben.

Es wurde nun gefunden, dass sich die zuvor aufgeführten Nachteile herkömmlicher Mischverfahren durch ein Verfahren zur Herstellung eines Färbemittels für Keratinfasern aus einer ersten Zusammensetzung A und einer zweiten Zusammensetzung B, beseitigen lassen, bei welchem
- eine Teilmenge a der ersten Zusammensetzung A aus einem Behälter A
- mittels einer Einfüllvorrichtung
- über eine Einlassöffnung
   in einen die zweite Zusammensetzung B enthaltenden zweiten Behälter B geleitet wird, dadurch gekennzeichnet, dass der Behälter B durch den infolge der Einleitung der Zusammensetzung A im Behälter B zunehmenden Druck und/oder durch den infolge der Penetration einer Behälterwand aufgrund der Einwirkung der Einfüllvorrichtung im Behälter B auftretenden Druck mindestens eine Austrittsöffnung ausbildet, aus welcher das Färbemittel für Keratinfasern als Mischung der Zusammensetzungen A und B aus dem Behälter B austritt,
- die im Behälter B befindliche Zusammensetzung B im Laufe des Verfahrens mittels der eingeleiteten Zusammensetzung A durch die Austrittsöffnung aus dem Behälter B ausgetragen wird und
- eine Restmenge der in dem Behälter A befindlichen Zusammensetzung A bis zum Ende des Mischverfahrens in der Behälter A verbleibt und diese Restmenge mindestens der doppelten Menge der Teilmenge a entspricht.

Im Rahmen des erfindungsgemäßen Verfahrens werden zwei voneinander verschiedene Zusammensetzungen A und B unter Ausbildung eines Färbemittels für Keratinfasern miteinander vermischt.

Die Zuleitung der Zusammensetzung A erfolgt aus einem Behälter A. Dieser Behälter A ist als Vorratsbehälter ausgestaltet und umfasst bevorzugt die mehrfache für die Durchführung eines einzelnen Mischverfahrens notwendige Menge der Zusammensetzung A. Mit anderen Worten wird eine Teilmenge a der in dem Behälter A befindlichen Zusammensetzung A in den Behälter B eingeleitet, wobei eine Restmenge der in dem Behälter A befindlichen Zusammensetzung A bis zum Ende des Mischverfahrens in dem Behälter A verbleibt und diese Restmenge mindestens der doppelten, vorzugsweise mindestens der vierfachen und insbesondere mindestens der achtfachen Menge der Teilmenge a entspricht.

In einer weiteren bevorzugten Ausführungsform weist der Behälter A zwei oder mehr Kammern (z.B.: A1 und A2) auf, in denen voneinander verschiedene Zusammensetzungen (z.B.: A1 und A2) voneinander getrennt vorliegen. Die zur Durchführung des erfindungsgemäßen Verfahrens eingesetzte Vorrichtung ist dabei derart ausgestaltet, dass durch den Anwender zwischen den zwei oder mehr Kammern gewählt und alternativ beispielsweise in einem ersten Mischverfahren eine Zusammensetzung A1 und in dem nachfolgenden Mischverfahren eine Zusammensetzung A2 eingesetzt wird.

Alternativ zu der zuvor beschriebenen Mehrkammerausführung des Behälters A kann die Vorrichtung zur Durchführung des erfindungsgemäßen Verfahrens auch zwei oder mehr separate Behälter für die Aufnahme unterschiedlicher Zusammensetzungen A vorsehen.

Die Einleitung der Zusammensetzung A aus dem Behälter A in den Behälter B erfolgt vorzugsweise mittels einer an den Behälter A anschließenden Leitungssystems. Am Ende dieses Leitungssystems befindet sich die zur Einleitung der Zusammensetzung A in den Behälter B vorgesehene Einfüllvorrichtung. Zur Verkürzung der Verfahrensdauer und zur Verbesserung des Verfahrensergebnisses, insbesondere der Güte der Vermischung, wird die Zusammensetzung A vorzugsweise mit einem Druck oberhalb 1,1 bar, vorzugsweise oberhalb 2,0 bar, bevorzugt oberhalb 5,0 bar und insbesondere zwischen 10 und 20 bar in den Behälter B eingeleitet.

Die Zusammensetzung A wird im Laufe des Verfahrens in den Behälter B eingeleitet, aus welchem nachfolgend das Färbemittel für Keratinfasern als Mischung der Zusammensetzungen A und B aus dem Behälter B austritt.

Der hierfür eingesetzte Behälter B wird vorzugsweise mittels eines Haft-, Rast-, Schnapp- oder Klemmmechanismus in der zur Durchführung des erfindungsgemäßen Verfahrens eingesetzten Vorrichtung befestigt.

Der Behälter B ist vorzugsweise in Form einer verschlossenen Kapsel ausgeführt. Diese verschlossene Kapsel wird in einer bevorzugten Variante des erfindungsgemäßen Verfahrens mittels der, die Zusammensetzung A führenden Leitungssystems geöffnet. Der Öffnungsvorgang erfolgt durch Penetration der Behälterwand des Behälters B mittels der am Ende des Leitungssystems befindlichen Einfüllvorrichtung. Diese Einfüllvorrichtung kann beispielsweise in Form eines Doms ausgebildet sein. Nach Durchstoßen der Behälterwand wird die Zusammensetzung A dann in den Behälter B eingeleitet.

In dem erfindungsgemäßen Verfahren wird durch die Einleitung der Zusammensetzung A im Behälter B mindestens eine Austrittsöffnung ausgebildet. Ursache für die Ausbildung der Austrittsöffnung ist der in dem Behälter B zunehmende Druck. Alternativ kann die Austrittsöffnung jedoch auch durch die Einwirkung der Einfüllvorrichtung ausgebildet werden, indem der in dem Behälter durch die Penetration einer Behälterwand auftretende Druck die Bildung der Austrittsöffnung bewirkt.

Die Ausbildung der Austrittsöffnung in der Behälterwand des Behälters B, insbesondere der exakte Ort, an welchem diese Austrittsöffnung ausgebildet wird, wird vorzugsweise durch die spezifische Konstruktion des Behälters B gesteuert.

In einer ersten bevorzugten Ausführungsform verfügt der Behälter B über eine Schwächungslinie, entlang derer sich durch die Einleitung der Zusammensetzung A und/oder die Einwirkung der Einfüllvorrichtung die Austrittöffnung ausbildet.

In einer zweiten bevorzugten Ausführungsform verfügt der Behälter über eine Membran, welche durch die Einleitung der Zusammensetzung A und/oder die Einwirkung der Einfüllvorrichtung unter Ausbildung der Austrittöffnung gegen einen Dorn gedrückt wird. Die Membran ist vorzugsweise Bestandteil der Behälterwand des Behälters B. Durch die Penetration der Membran mittels des Dorns wird die Austrittsöffnung erzeugt. Der Dorn kann dabei sowohl innerhalb des Behälters B, als auch außerhalb des Behälters B angeordnet sein. Bei einem innerhalb des Behälters B angeordneten Dorn wird die Behälterwand des Behälters B von innen nach außen geöffnet. Befindet sich der Dorn außerhalb des Behälters B, drückt der Dorn die Behälterwand von außen nach innen. Vorzugsweise weist die Behälterwand des Behälters B im Einwirkbereich des Dorn eine Schwächungslinie auf, durch welche beispielsweise die Größe der Austrittsöffnung beeinflusst werden kann.

Zur Verbesserung der Mischwirkung durchläuft die Zusammensetzung A und/oder das Gemisch der Zusammensetzungen A und B im Verlauf des Verfahrens vorzugsweise einen statischen Mischer. Dieser statische Mischer kann beispielsweise innerhalb des oben beschriebenen Leitungssystems angeordnet sein, befindet sich jedoch vorzugsweise in unmittelbarer Nähe der Austrittsöffnung des Behälters B, beispielsweise innerhalb des Behälters B oder außerhalb der Austrittsöffnung. Im letztgenannten Fall kann der statische Mischer als integraler Bestandteil des Behälters B ausgebildet sein. Alternativ ist der statische Mischer Bestandteil der zur Durchführung des erfindungsgemäßen Verfahrens eingesetzten Vorrichtung und ist beispielsweise dem zur Befestigung des Behälters B eingesetzten Haft-, Rast-, Schnapp- oder Klemmmechanismus zugeordnet.

Die im Behälter B befindliche Zusammensetzung B wird im Laufe des Verfahrens mittels der eingeleiteten Zusammensetzung A durch die Austrittsöffnung aus dem Behälter B ausgetragen. Der Austrag erfolgt dabei vorzugsweise im Wesentlichen vollständig. Mit anderen Worten wird die Zusammensetzung B aus dem Behälter zu mindestens 80 Gew.-%, vorzugsweise zu mindestens 90 Gew.-%, besonders bevorzugt zu mindestens 95 Gew.-% und insbesondere zu mindestens 98 Gew.-% ausgetragen.

Das Volumenverhältnis der in dem Mischverfahren eingesetzten Zusammensetzungen A und B beträgt vorzugsweise 10:1 bis 1:1 und insbesondere 6:1 bis 2:1. Das absolute Volumen der eingesetzten Zusammensetzung A beträgt bevorzugt zwischen 5 und 500 ml, vorzugsweise zwischen 10 und 400 ml und insbesondere zwischen 20 und 300 ml.

Die Zusammensetzungen A und B werden im Verlauf des Mischverfahrens vorzugsweise nicht durch eine äußere Wärmequelle erwärmt. Die Temperatur der Zusammensetzung A sollte vorzugsweise weniger als 35°C, bevorzugt weniger als 30°C und insbesondere weniger als 25°C betragen. Die Temperatur des Färbemittels für Keratinfasern sollte bei Austritt aus dem Behälter B ebenfalls vorzugsweise weniger als 35°C, bevorzugt weniger als 30°C und insbesondere weniger als 25°C betragen.

Die in dem erfindungsgemäßen Verfahren miteinander vermischten Zusammensetzungen A und B können eine Vielzahl von haarfarbverändernden Wirkstoffen enthalten. So können beispielsweise
- zwei verschiedene oxidative Färbemittel,
- zwei verschiedene Tönungsmittel,
- zwei verschieden starke Blondiermittel,
- ein Blondiermittel und ein oxidatives Färbemittel,
- ein Blondiermittel und ein Tönungsmittel oder
- ein oxidatives Färbemittel und ein Tönungsmittel
in dem erfindungsgemäßen Verfahren zu einem Färbemittel für Keratinfasern vermischt werden.

Die Zusammensetzung A ist vorzugsweise fließfähig und liegt in Form einer Flüssigkeit, eines Gels oder einer Paste vor. Besonders bevorzugte flüssige Zusammensetzungen A enthalten mindestens 30 Gew.-%, vorzugsweise mindestens 40 Gew.-% und insbesondere mindestens 50 Gew.-% Wasser. Der Gewichtsanteil des Wassers beträgt vorzugsweise zwischen 30 und 98 Gew.-%, bevorzugt zwischen 40 und 96 Gew.-% und insbesondere zwischen 50 und 94 Gew.-%, jeweils bezogen auf das Gesamtgewicht der Zusammensetzung A.

In einer bevorzugten Verfahrensvariante enthält die Zusammensetzung A mindestens ein Oxidationsmittel, vorzugsweise 0,5 bis 50 Gew.-%, bevorzugt 1,0 bis 20 Gew.-%, besonders bevorzugt 2,5 bis 16 Gew.-% und insbesondere 5,0 bis 14 Gew.-% Wasserstoffperoxid (berechnet als 100%iges H₂O₂), jeweils bezogen auf das Gesamtgewicht der Zusammensetzung A, enthält.

Zur Einstellung der Viskosität kann die Zusammensetzung A ein Verdickungsmittel enthalten, wobei vorzugsweise 1,0 bis 30 Gew.-%, bevorzugt 3,0 bis 25 Gew.-%, besonders bevorzugt 5,0 bis 20 Gew.-% mindestens eines Homo- oder Copolymers, das gebildet wird aus einem Monomerengemisch von ethylenisch ungesättigten Säuren und/oder deren einfachen C₁- bis C₆-Alkylestern, jeweils bezogen auf das Gesamtgewicht der Zusammensetzung A, eingesetzt werden.

Die Zusammensetzung B kann in fließfähiger Form, beispielsweise als Flüssigkeit, Gel oder Paste, aber auch als Feststoff, insbesondere als Pulver oder verpresstes Pulver vorliegen. Hinsichtlich der Verfahrensdauer und zur Verbesserung des Verfahrensergebnisses, insbesondere der Güte der Vermischung, haben sich jedoch fließfähige gegenüber festen Zusammensetzungen B als erwiesen.

Die erfindungsgemäßen Verfahren dienen der einfachen und effizienten Herstellung von Färbemitteln für keratinische Fasern. Entsprechende Mittel enthalten demnach naturgemäß geeignete färbende oder entfärbende Aktivsubstanzen. Bevorzugte Verfahrensvarianten sind dadurch gekennzeichnet, dass die Zusammensetzung B mindestens ein Oxidationsfarbstoffvorprodukt oder mindestens einen direktziehenden Farbstoff enthält.

In einer ersten bevorzugten Ausführungsform enthält die Zusammensetzung B mindestens eine oxidatives Färbemittel (Oxidationsfarbstoffvorprodukt).

Unter oxidativen Färbemittel sind erfindungsgemäß haarfarbverändernde Mittel zu verstehen, die durch Oxidation von Oxidationsfarbstoffvorprodukten eine dauerhafte Färbung der Fasern bewirken.

Hinsichtlich der in den erfindungsgemäßen Zusammensetzungen B einsetzbaren Farbstoffvorprodukte unterliegt die vorliegende Erfindung keinerlei Einschränkungen. Die erfindungsgemäßen Zusammensetzungen B können als Farbstoffvorprodukte Oxidationsfarbstoffvorprodukte vom Entwickler- und/oder Kuppler-Typ, und Vorstufen naturanaloger Farbstoffe, wie Indol- und Indolin-Derivate, sowie Mischungen von Vertretern dieser Gruppen enthalten.

Im Rahmen einer ersten bevorzugten Ausführungsform der vorliegenden Erfindung enthalten die erfindungsgemäß eingesetzten Zusammensetzungen B mindestens ein Oxidationsfarbstoffvorprodukt vom Entwickler- und/oder Kupplertyp.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 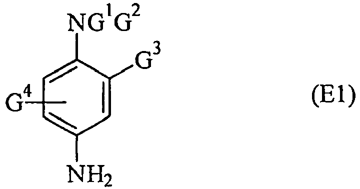 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-kytendioxogruppe, wie beispielsweise eine Ethylendioxygruppe bilden.

Beispiele für die als Substituenten in den erfindungsgemäßen Verbindungen genannten C₁-bis C₄-Alkylreste sind die Gruppen Ethyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, C₁- oder Br-Atome, C₁-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 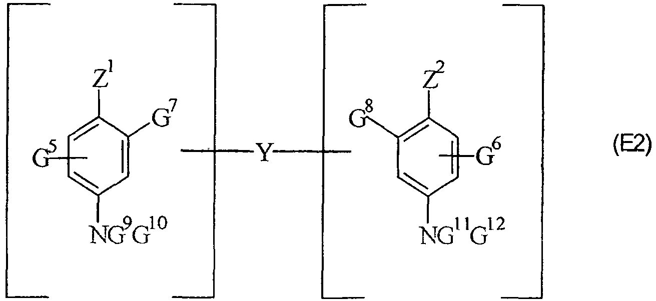 wobei:
1. Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
   - die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
   - G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
   - G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 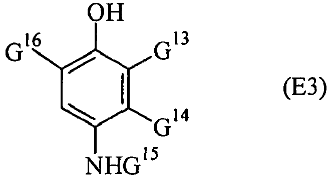 wobei:
- G¹³ steht für ein Wasserstoffatom, ein Halogenatom, einen C₁- bis C₄-Alkylrest, einen C₁-bis C₄-Monohydroxyalkylrest, einen C₂- bis C₄-Polyhydroxyalkylrest, einen (C₁- bis C₄)-Alkoxy-(C₁-bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-l-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 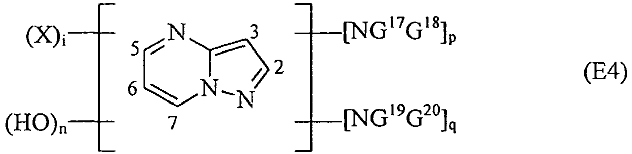 wobei:
- G¹⁷, G¹⁸, G¹⁹ und G²⁰ unabhängig voneinander stehen für ein Wasserstoffatom, einen C₁-bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁-bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁-bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

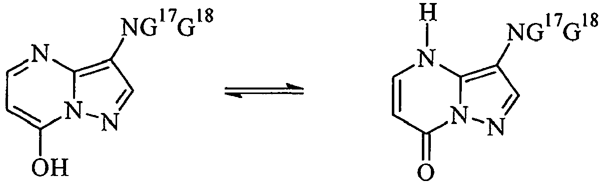

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin:
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die Zusammensetzungen B mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
(A) m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
(B) o-Aminophenol und dessen Derivate,
(C) m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
(D) o-Diaminobenzyl und dessen Derivate wie beispielsweise 3,4-Diaminobenzoesäure und 2,3-Diamino-1-methylbenzol,
(E) Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
(F) Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
(G) Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
(H) Morpholinderivate wie beispielsweise 6-Hydroxybenzomorpholin und 6-Amino-benzomorpholin,
(I) Chinoxalinderivate wie beispielsweise 6-Methyl-1,2,3,4-tetrahydrochinoxalin,
(J) Pyrazolderivate wie beispielsweise 1-Phenyl-3-methylpyrazol-5-on,
(K) Indolderivate wie beispielsweise 4-Hydroxyindol, 6-Hydroxyindol und 7-Hydroxyindol,
(L) Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
(M) Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Die erfindungsgemäß eingesetzten Zusammensetzungen B enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das Gesamtgewicht der Zusammensetzung BI. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

In einer weiteren Ausführungsform der vorliegenden Erfindung enthalten die Zusammensetzungen B als Oxidationsfarbstoffvorprodukt mindestens eine Vorstufe eines naturanalogen Farbstoffs. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Zusammensetzungen B mindestens ein Indol- und/oder Indolinderivat.

Besonders gut als Vorstufen naturanaloger Haarfarbstoffe geeignet sind Derivate des 5,6-Dihydroxyindolins der Formel (IIa), 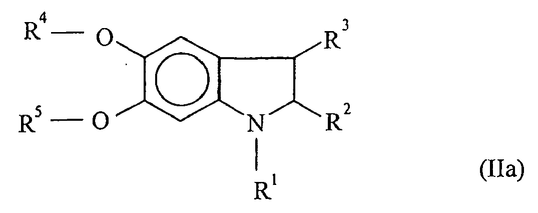 in der unabhängig voneinander
1. R¹ steht für Wasserstoff, eine C₁-C₄-Alkylgruppe oder eine C₁-C₄-Hydroxy-alkylgruppe,
2. R² steht für Wasserstoff oder eine -COOH-Gruppe, wobei die -COOH-Gruppe auch als Salz mit einem physiologisch verträglichen Kation vorliegen kann,
3. R³ steht für Wasserstoff oder eine C₁-C₄-Alkylgruppe,
4. R⁴ steht für Wasserstoff, eine C₁-C₄-Alkylgruppe oder eine Gruppe -CO-R⁶, in der R⁶ steht für eine C₁-C₄-Alkylgruppe, und
5. R⁵ steht für eine der unter R⁴ genannten Gruppen,
   sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbon-säure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindol der Formel (IIb), 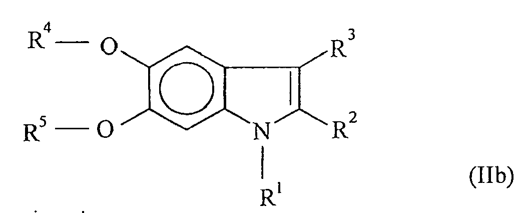 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäß eingesetzten Zusammensetzungen B sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in den Zusammensetzungen B in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

In einer zweiten bevorzugten Ausführungsform enthält die Zusammensetzung B mindestens einen direktziehenden Farbstoff.

Neben den Oxidationsfarbstoffvorprodukten oder alternativ zu diesen Färbemitteln können die Zusammensetzungen B auch direktziehende Farbstoffe enthalten.

Die direktziehenden Farbstoffe sind vorzugsweise ausgewählt aus den Nitrophenylendiaminen, den Nitroaminophenolen, den Azofarbstoffen, den Anthrachinonen oder den Indophenolen.

Besonders bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner kann es erfindungsgemäß bevorzugt sein, dass die Zusammensetzungen B mindestens einen kationischen direktziehenden Farbstoff enthalten. Besonders bevorzugt sind dabei
1. kationische Triphenylmethanfarbstoffe, wie beispielsweise Basic Blue 7, Basic Blue 26, Basic Violet 2 und Basic Violet 14,
2. aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
3. direktziehende Farbstoffe, die mindestens einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

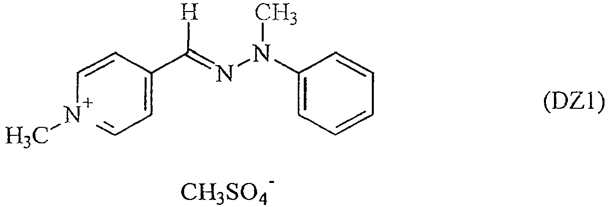

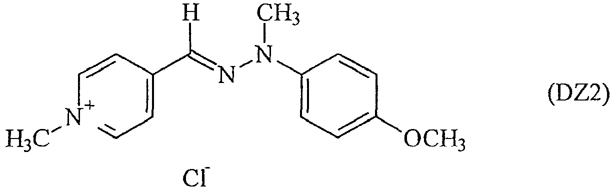

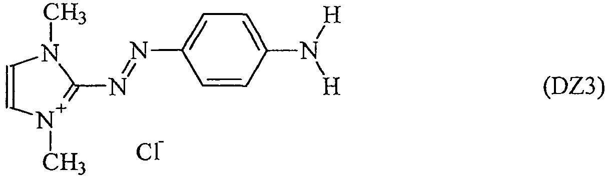

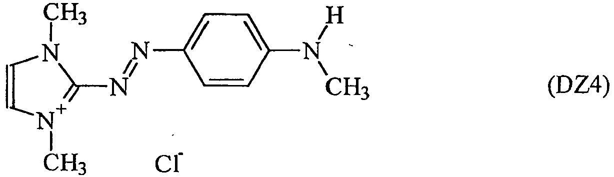

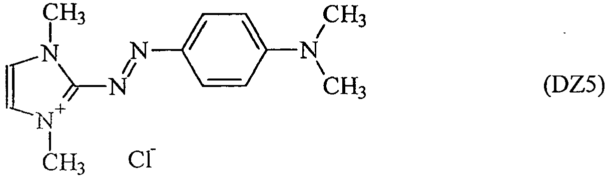

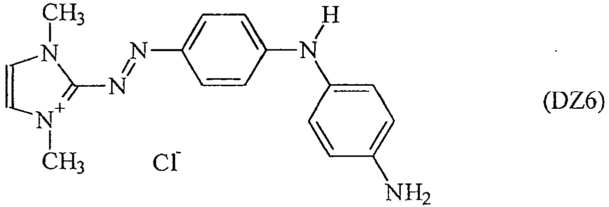

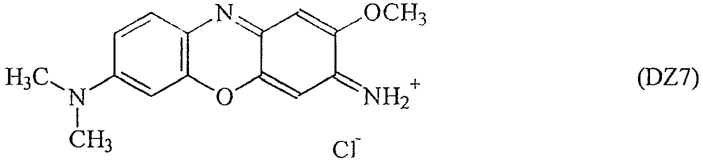

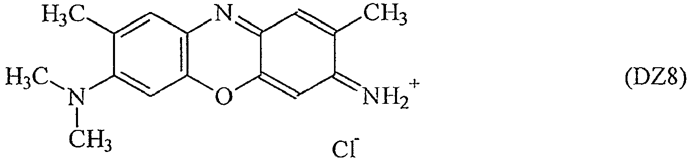

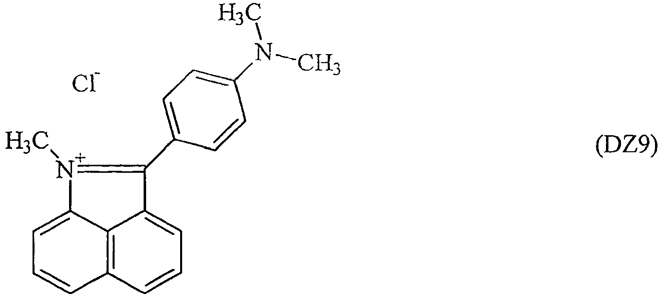

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Weiterhin können die Zusammensetzungen B auch in der Natur vorkommende direktziehende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Die Zusammensetzungen B enthalten die direktziehenden Farbstoffe bevorzugt in einer Menge von 0,01 bis 20 Gew.-%, bezogen auf die gesamten Anwendungszubereitung in dem jeweiligen Behälter.

In einer dritten bevorzugten Ausführungsform wird als Zusammensetzung B ein Blondiermittel, vorzugsweise ein Blondierpulver eingesetzt. Zur Erzeugung der Blondierwirkung enthalten diese Blondiermittel vorzugsweise sogenannte "Booster". Dies sind in der Regel feste Peroxoverbindungen, die keine Anlagerungsprodukte von Wasserstoffperoxid an andere Komponenten darstellen. Die Auswahl dieser Peroxoverbindungen unterliegt prinzipiell keinen Beschränkungen; übliche, dem Fachmann bekannte Peroxoverbindungen sind beispielsweise Ammoniumperoxodisulfat, Kaliumperoxodisulfat, Natriumperoxodisulfat, Ammoniumpersulfat, Kaliumpersulfat, Natriumpersulfat, Kaliumperoxodiphosphat, Percarbonate wie Magnesiumpercarbonat und Peroxide wie Bariumperoxid. Unter diesen Peroxoverbindungen, die auch in Kombination eingesetzt werden können, sind erfindungsgemäß die anorganischen Verbindungen bevorzugt. Besonders bevorzugt sind die Peroxodisulfate, insbesondere Ammoniumperoxodisulfat.

Die Peroxoverbindungen sind in den erfindungsgemäß als Zusammensetzung B eingesetzten Blondiermitteln bevorzugt in Mengen von 2-50 Gew.-%, insbesondere in Mengen von 10-35 Gew.-%, enthalten. Erfindungsgemäß bevorzugte Verfahren sind dadurch gekennzeichnet, dass die Zusammensetzung B mindestens ein Oxidationsmittel, vorzugsweise 5,0 bis 50 Gew.-%, bevorzugt 10 bis 45 Gew.-%, besonders bevorzugt 15 bis 40 Gew.-% und insbesondere 20 bis 35 Gew.-% Persulfat, jeweils bezogen auf das Gesamtgewicht der Zusammensetzung B, enthält.

Als weitere wichtige Komponente enthalten die erfindungsgemäß eingesetzten Blondiermittel ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxide, - carbonate, -hydrogencarbonate, -hydroxycarbonate, -silikate, insbesondere -metasilikate, sowie Alkaliphosphate verwendet werden. In einer bevorzugten Ausführungsform enthalten die erfindungsgemäßen Blondiermittel mindestens zwei unterschiedliche Alkalisierungsmittel. Dabei können Mischungen beispielsweise aus einem Hydroxycarbonat und einem Metasilikat bevorzugt sein.

Der Gewichtsanteil des Alkalisierungsmittels am Gesamtgewicht des als Zusammensetzung B eingesetzten Blondiermittels beträgt vorzugsweise 5 bis 50 Gew.-%, bevorzugt 10 bis 45 Gew.-% und insbesondere 12 bis 40 Gew.-%.

Wird als Zusammensetzung B ein Blondiermittel eingesetzt, liegt dieses vorzugsweise in Pulverform vor, wobei in der Regel zusätzlich eine Komponente zur Entstaubung der feinpulverisierten Formulierung zugesetzt wird. Solche Entstaubungsmittel sind üblicherweise Öle, flüssige Wachse, Etherderivate aber auch bei 25°C flüssige Lösemittel, ausgewählt aus der Gruppe der Kohlenwasserstoffe, der Alkohole, der Ester sowie der Ketone, wie z.B. 3-Methoxybutanol, Benzylalkohol, 1,2-Propandiol, Hexanol, Cyclohexanon, Propylencarbonat und Ethyldiglykol.

Zur Einstellung der Viskosität kann die Zusammensetzung B ein Verdickungsmittel enthalten, wobei insbesondere feste Zusammensetzungen B, insbesondere feste, bleichmittelhaltige Zusammensetzungen B vorzugsweise 0,5 bis 20 Gew.-%, bevorzugt 1,0 bis 15 Gew.-%, besonders bevorzugt 1,5 bis 10 Gew.-% Xanthan und/oder Carboxycellulose enthalten.

Die erfindungsgemäß hergestellten Färbemittel bzw. die zur Herstellung eingesetzten Zusammensetzungen A und B können weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinyl-pyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-tri-methylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten.

Bevorzugte erfindungsgemäße Verfahren dienen der Herstellung von Färbemitteln für Keratinfasern mit einer Viskosität von 10000 bis 100000 mPas, vorzugsweise von 30000 bis 50000 mPas (Brookfield RVF Viskosimeter, Spindel 4. 20U/min, 20°C) beträgt.

Das als Verfahrensendprodukt erhaltene Färbemittel für Keratinfasern weist vorzugsweise einen pH-Wert zwischen 5 und 12, vorzugsweise zwischen 7,5 und 11 auf.

Wie eingangs ausgeführt, dient das erfindungsgemäße Verfahren insbesondere der Herstellung von Färbemitteln für das menschliche Haar. Erfindungsgemäß bevorzugte Verfahren sind daher dadurch gekennzeichnet, dass das Färbemittel für Keratinfasern nach Austritt aus dem Behälter B auf Keratinfasern, vorzugsweise menschliches Haar, aufgebracht wird. Die Auftragung des Färbemittels erfolgt dabei vorzugsweise unmittelbar, das heißt innerhalb eines Zeitraum von weniger als 30 Minuten, bevorzugt von weniger als 15 Minuten, besonders bevorzugt von weniger als 10 Minuten und insbesondere von weniger als 5 Minuten.

Das Volumen der vorgenannten Behälter beträgt vorzugsweise 5 bis 100 ml, bevorzugt 10 bis 80 ml und insbesondere 20 bis 60 ml.

Bevorzugte Behälter weisen eine zylindrische Mantelfläche, eine plane Oberseite sowie eine dieser Oberseite gegenüberliegende plan oder konisch ausgeführte Unterseite auf. Besonders bevorzugte Behälter weisen eine die Flansch auf, an welchem eine den Behälter verschließende Siegelfolie befestigt ist. Ein solcher Flansch erleichtert beispielsweise die Befestigung des Behälters mittels eines Haft-, Rast-, Schnapp- oder Klemmmechanismus in der zur Durchführung des erfindungsgemäßen Verfahrens eingesetzten Vorrichtung.

Die vorgenannten Behälter werden vorzugsweise aus chemisch inerten Materialien gefertigt. Zur Gruppe dieser Materialien zählen beispielsweise Aluminium oder Kunststoffe wie Polypropylen.

## Patentansprüche

1. Verfahren zur Herstellung eines Färbemittels für Keratinfasern aus einer ersten Zusammensetzung A und einer zweiten Zusammensetzung B, bei welchem
- eine Teilmenge a der ersten Zusammensetzung A aus einem Behälter A
- mittels einer Einfüllvorrichtung
- über eine Einlassöffnung
- in einen die zweite Zusammensetzung B enthaltenden zweiten Behälter B geleitet wird,
**dadurch gekennzeichnet, dass**
- der Behälter B durch den infolge der Einleitung der Zusammensetzung A im Behälter B zunehmenden Druck und/oder durch den infolge der Penetration einer Behälterwand aufgrund der Einwirkung der Einfüllvorrichtung im Behälter B auftretenden Druck mindestens eine Austrittsöffnung ausbildet, aus welcher das Färbemittel für Keratinfasern als Mischung der Zusammensetzungen A und B aus dem Behälter B austritt,
- die im Behälter B befindliche Zusammensetzung B im Laufe des Verfahrens mittels der eingeleiteten Zusammensetzung A durch die Austrittsöffnung aus dem Behälter B ausgetragen wird und
- eine Restmenge der in dem Behälter A befindlichen Zusammensetzung A bis zum Ende des Mischverfahrens in der Behälter A verbleibt und diese Restmenge mindestens der doppelten Menge der Teilmenge a entspricht.

2. Verfahren nach Anspruch 1, **dadurch gekennzeichnet, dass** die Zusammensetzung A mit einem Druck oberhalb 1,1 bar, vorzugsweise oberhalb 2,0 bar, bevorzugt oberhalb 5,0 bar und insbesondere zwischen 10 und 20 bar in den Behälter B eingeleitet wird.

3. Verfahren nach einem der Ansprüche 1 oder 2, **dadurch gekennzeichnet, dass** der Behälter B über eine Schwächungslinie verfügt, entlang derer sich durch die Einleitung der Zusammensetzung A und/oder die Einwirkung der Einfüllvorrichtung die Austrittöffnung ausbildet.

4. Verfahren nach einem der vorherigen Ansprüche, **dadurch gekennzeichnet, dass** der Behälter über eine Membran verfügt, welche durch die Einleitung der Zusammensetzung A und/oder die Einwirkung der Einfüllvorrichtung unter Ausbildung der Austrittöffnung gegen einen Dorn gedrückt wird.

5. Verfahren nach einem der vorherigen Ansprüche, **dadurch gekennzeichnet, dass** der Behälter B in seinem Inneren ein statisches Mischelement aufweist.

6. Verfahren nach einem der vorherigen Ansprüche, **dadurch gekennzeichnet, dass** die die Zusammensetzung A ein Oxidationsmittel, vorzugsweise 0,5 bis 50 Gew.-%, bevorzugt 1,0 bis 20 Gew.-%, besonders bevorzugt 2,5 bis 16 Gew.-% und insbesondere 5,0 bis 14 Gew.-% Wasserstoffperoxid (berechnet als 100%iges H₂O₂), jeweils bezogen auf das Gesamtgewicht der Zusammensetzung A, enthält.

7. Verfahren nach einem der vorherigen Ansprüche, **dadurch gekennzeichnet, dass** die Zusammensetzung B mindestens ein Oxidationsfarbstoffvorprodukt oder mindestens einen direktziehenden Farbstoff enthält.

8. Verfahren nach einem der vorherigen Ansprüche, **dadurch gekennzeichnet, dass** die Zusammensetzung B mindestens ein Oxidationsmittel, vorzugsweise 5,0 bis 50 Gew.-%, bevorzugt 10 bis 45 Gew.-%, besonders bevorzugt 15 bis 40 Gew.-% und insbesondere 20 bis 35 Gew.-% Persulfat, jeweils bezogen auf das Gesamtgewicht der Zusammensetzung A, enthält.

9. Verfahren nach einem der vorherigen Ansprüche, **dadurch gekennzeichnet, dass** die Zusammensetzungen A und B in einem Volumenverhältnis von 10:1 bis 1:1, vorzugsweise von 6:1 bis 2:1 eingesetzt werden.

10. Verfahren nach einem der vorherigen Ansprüche, **dadurch gekennzeichnet, dass** das haarfarbverändernde Mittel nach Austritt aus dem Behälter B auf das Haar aufgebracht wird.

## Claims

1. A method for producing a dye for keratin fibers from a first composition A and a second composition B, in which
- a partial quantity a of the first composition A is guided out of a container A
- by means of a filling device
- through an inlet opening
- into a container B containing the second composition B,
**characterized in that**
- at least one outlet opening is formed in the container B from which the dye for keratin fibers leaves the container B as a mixture of compositions A and B as a result of the increasing pressure in the container B due to the introduction of composition A and/or as a result of the pressure in the container B due to the penetration of a container wall because of the action of the filling device,
- over the course of the method, the composition B in the container B is discharged from the container B through the outlet opening by means of the introduced composition A and
- a remaining quantity of composition A in the container A remains in the container A until the end of the mixing process, and this remaining quantity corresponds to at least double the partial quantity a.

2. The method according to claim 1, **characterized in that** composition A is introduced into the container B at a pressure of above 1.1 bar, preferably of above 2.0 bar, more preferably of above 5.0 bar and in particular of between 10 and 20 bar.

3. The method according to one of claims 1 or 2, **characterized in that** the container B has a line of weakness, along which the outlet opening is formed as a result of the introduction of composition A and/or the action of the filling device.

4. The method according to one of the preceding claims, **characterized in that** the container has a membrane which, as a result of the introduction of composition A and/or the action of the filling device, is pressed against a spike so as to form the outlet opening.

5. The method according to one of the preceding claims, **characterized in that** the container B comprises a static mixing element in its interior.

6. The method according to one of the preceding claims, **characterized in that** composition A contains an oxidizing agent, preferably 0.5 to 50 wt.%, more preferably 1.0 to 20 wt.%, particularly preferably 2.5 to 16 wt.% and in particular 5.0 to 14 wt.% hydrogen peroxide (calculated as 100% H₂O₂), in each case based on the total weight of composition A.

7. The method according to one of the preceding claims, **characterized in that** composition B contains at least one oxidation dye precursor or at least one direct dye.

8. The method according to one of the preceding claims, **characterized in that** composition B contains at least one oxidizing agent, preferably 5.0 to 50 wt.%, more preferably 10 to 45 wt.%, particularly preferably 15 to 40 wt.% and in particular 20 to 35 wt.% persulfate, in each case based on the total weight of composition A.

9. The method according to one of the preceding claims, **characterized in that** compositions A and B are used in a volume ratio of from 10:1 to 1:1, preferably of from 6:1 to 2:1.

10. The method according to one of the preceding claims, **characterized in that** the hair-color-changing agent is applied to the hair after leaving the container B.

## Revendications

1. Procédé de préparation d'un colorant, destiné à des fibres de kératine, qui est constitué d'une première composition A et d'une seconde composition B, procédé dans lequel
- une quantité a de la première composition A est amenée d'un récipient A
- au moyen d'un dispositif de remplissage,
- par un orifice d'entrée
- jusque dans un seconde récipient B contenant la seconde composition B,
**caractérisé en ce que**
- en raison de la pression croissante dans le récipient B due à l'introduction de la composition A et/ou en raison de la pression générée dans le récipient B à cause de la pénétration d'une paroi de récipient due à l'action du dispositif de remplissage, le récipient B forme au moins un orifice de sortie par lequel le colorant destiné aux fibres de kératine sort du récipient B sous forme de mélange des compositions A et B,
- la composition B placée dans le récipient B est évacuée du récipient B par l'orifice de sortie au cours du procédé au moyen de la composition A introduite et
- une quantité résiduelle de la composition A placée dans le récipient A reste dans le récipient jusqu'à la fin du processus de mélange et cette quantité résiduelle correspond au moins à deux fois la quantité a.

2. Procédé selon la revendication 1, **caractérisé en ce que** la composition A est introduite dans le récipient B à une pression supérieure à 1,1 bar, de préférence supérieure à 2,0 bar, préférablement supérieure à 5,0 bars et en particulier entre 10 et 20 bars.

3. Procédé selon l'une des revendications 1 ou 2, **caractérisé en ce que** le récipient B dispose une ligne de moindre résistance mécanique le long de laquelle l'ouverture de sortie se forme par l'introduction de la composition A et/ou l'action du dispositif de remplissage

4. Procédé selon l'une des revendications précédentes, **caractérisé en ce que** le récipient dispose d'une membrane qui est pressée contre une broche par l'introduction de la composition A et/ou l'action du dispositif de remplissage par formation de l'ouverture de sortie.

5. Procédé selon l'une des revendications précédentes, **caractérisé en ce que** le récipient B comporte à l'intérieur un élément de mélange statique.

6. Procédé selon l'une des revendications précédentes, **caractérisé en ce que** la composition A contient un agent oxydant, de préférence de 0,5 à 50% en poids, préférablement de 1,0 à 20% en poids, de façon particulièrement préférée de 2,5 à 16 poids% et en particulier de 5,0 à 14% en poids, de peroxyde d'hydrogène (calculé sur 100% de H₂O₂), à chaque fois par rapport au poids total de la composition A.

7. Procédé selon l'une des revendications précédentes, **caractérisé en ce que** la composition B comprend au moins un précurseur de colorant d'oxydation ou au moins un colorant direct.

8. Procédé selon l'une des revendications précédentes, **caractérisé en ce que** la composition B contient au moins un agent oxydant, de préférence de 5,0 à 50% en poids, de préférence de 10 à 45% en poids, de manière particulièrement préférée de 15 à 40% en poids et en particulier de 20 à 35% en poids, de persulfate, à chaque fois par rapport au poids total de la composition A.

9. Procédé selon l'une des revendications précédentes, **caractérisé en ce que** les compositions A et B sont utilisées dans un rapport en volume de 10:1 à 1:1, de préférence de 6:1 2:1.

10. Procédé selon l'une des revendications précédentes, **caractérisé en ce que** l'agent de changement de couleur de cheveux est appliqué sur les cheveux après la sortie du récipient B.
